# EUROPÄISCHE PATENTSCHRIFT

(11) **EP 2 173 318 B1**
(45) Veröffentlichungstag und Bekanntmachung des Hinweises auf die Patenterteilung: **11.11.2015**
(21) Anmeldenummer: 08785962.5
(22) Anmeldetag: 07.07.2008
(51) Int. Cl.: A61K 8/892, A61Q 5/06, A61K 8/58

(54) **VERWENDUNG VON STYLINGMITTELN AUF BASIS WÄSSRIGER SILICONDISPERSIONEN ZUR REMODELLIERBAREN VERFORMUNG KERATINISCHER FASERN**
USE OF STYLING AGENTS BASED ON AQUEOUS SILICONE DISPERSIONS FOR REMODELLABLE STYLING OF KERATIN FIBRES
UTILISATION D'AGENTS DE MISE EN FORME À BASE DE DISPERSIONS AQUEUSES DE SILICONE POUR UNE MISE EN FORME REMODELABLE DES FIBRES KÉRATINIQUES

(30) Priorität: 08.08.2007 DE 102007037429
(43) Veröffentlichungstag der Anmeldung: 14.04.2010
(73) Patentinhaber: Henkel AG & Co. KGaA, 40589 Düsseldorf (DE)
(72) Erfinder: KRÜGERMANN, Ina, 40764 Langenfeld (DE); PLANTENBERG, Thomas, 51371 Leverkusen (DE); HELPENSTEIN, Klaus, 41199 Mönchengladbach (DE); WILLEMSEN, Yvonne, 41352 Korschenbroich (DE); KNAPPE, Thorsten, 22869 Schenefeld (DE)
(86) Internationale Anmeldenummer: PCT/EP2008/058792
(87) Internationale Veröffentlichungsnummer: WO 2009/019098

(56) Entgegenhaltungen:
- EP-A- 0 240 349
- EP-A- 0 410 899

## Beschreibung

Die vorliegende Erfindung betrifft die Verwendng von Mitteln, enthaltend in einem kosmetisch akzeptablen Träger mindestens eine spezielle wässrige Silicondispersion, zur remodellierbaren Verformung keratinischer Fasern.

Unter keratinischen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe oder gezielte Veränderung der Haarstruktur aufbauen bzw. für einen längeren Zeitraum aufrechterhalten lassen. Daher spielen Haarbehandlungsmittel, die einer permanenten oder temporären Formgebung der Haare dienen, eine wichtige Rolle.

Zur dauerhaften Verformung keratinhaltiger Fasern wird üblicherweise die Faser mechanisch verformt und die Verformung gegebenenfalls durch geeignete Hilfsmittel festgelegt. Vor und/oder nach dieser Verformung behandelt man die Faser mit einer keratinreduzierenden Zubereitung. Dabei wird ein Teil der Disulfid-Brücken des Keratin-Moleküls gespalten, so dass es zu einer Erweichung der Keratinfaser kommt. Nach einem Spülvorgang wird die Faser dann in dem sogenannten Fixierschritt mit einer Oxidationsmittelzubereitung behandelt, wobei erneut DisulfidBrücken im Haarkeratin geknüpft werden, so dass das Keratingefüge in der vorgegebenen Verformung fixiert wird. Es entsteht eine dauerhafte Verformung, die üblichen Umwelteinflüssen, wie beispielsweise dem Waschen der Haare, problemlos widersteht. Nachteilig an diesem Vorgehen ist jedoch, dass zwangsläufig die natürliche Struktur der behandelten Fasern verändert wird. Dies führt oft zu einer Schwächung der Fasern. Insbesondere bei wiederholter Dauerverformung oder der Durchführung weiterer die Faser belastender Behandlungen, wie beispielsweise oxidativen Färbungen oder Blondierungen, sind Schädigungen der Fasern nicht auszuschließen. Zudem ist es ohne eine erneute dauerhafte Verformung oder zumindest die Anwendung temporärer Stylingmittel nicht möglich, die einmal festgelegte Form zu verändern. Der Verbraucher wünscht sich aber gerade eine Möglichkeit der Frisurgestaltung, die das Haar nicht schädigt und zudem eine gewisse Flexibilität bietet.

Eine schonende Methode der Verformung keratinhaltiger Fasern sind die temporären Formgebungen, wie sie beispielsweise durch herkömmliche Haarsprays, Haarwachse, Haargele, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Eine wesentliche Eigenschaft eines Mittels zur Verformung keratinischer Fasern, im Folgenden auch Stylingmittel genannt, besteht darin, der behandelten Faser in der erzeugten Form einen möglichst starken Halt zu geben. Handelt es sich bei den keratinischen Fasern um menschliche Haare, spricht man auch von starkem Frisurenhalt. Darüber hinaus erwartet der Anwender von einem Stylingmittel in zunehmendem Maß, dass die erstellte Frisur verformbar bleibt ohne dass der Halt durch die Umformung verloren geht. Man spricht in diesem Zusammenhang auch von der Remodellierbarkeit einer Frisur. Auch die Feuchtebeständigkeit spielt oft eine Rolle. Die erstellte Frisur soll auch gegenüber hoher Luftfeuchtigkeit und Schweiß beständig sein.

Verfügbare Stylingmittel, die der Frisur guten Halt verleihen, basieren in der Regel auf filmbildenden und/oder festigenden Polymeren. Die Stärke des Halts lässt sich durch geeignete Wahl der Art und/oder Konzentration des filmbildenden und/oder festigenden Polymers einstellen. Die Polymere bilden auf der behandelten Faser einen Film aus und vernetzen einzelne Fasern miteinander, so dass die Fasern in einer vorgegebenen Position fixiert werden. Wird eine einmal fixierte Frisur mechanischer Belastung unterworfen, so kommt es leicht zum Bruch der Polymerfilme, Vernetzungsstellen werden irreversibel gelöst und der Halt der Fasern geht verloren. Da eine Umformung der Frisur zwangsläufig mit einer mechanischen Belastung einhergeht, lassen solche Stylingmittel keine Remodellierbarkeit der Frisur zu.

Verfügbare Stylingmittel, die eine gewisse Remodellierbarkeit erlauben, etwa Haarwachse oder entsprechende Pasten, basieren in der Regel auf Wachsen und weisen den Nachteil auf, dass der erzielbare Haltegrad gering ist.

Zudem lassen sich mit den bekannten Mitteln zur temporären Formgebung keine dauerhaften Effekte erzielen. Bei einer herkömmlichen Haarwäsche werden die Mittel ausgewaschen. Eine erneute Formgebung erfordert wiederum den Einsatz entsprechender Stylingmittel.

Der Einsatz verschiedener Silicone und Silicondispersionen in kosmetischen Mitteln ist prinzipiell bekannt, wobei diese Verbindungsklassen in der Regel wegen ihrer pflegenden Eigenschaften eingesetzt werden. Der Einsatz in Stylingmitteln, mit denen starker Halt erreicht werden soll, ist die Ausnahme, da sich viele Silicone nachteilig auf die festigenden Eigenschaften der Polymere auswirken, die üblicherweise in Stylingmitteln eingesetzt werden.

Ein Beispiel für den Einsatz bestimmter Siliconelastomere in wässrigen Aerosolstylingschäumen offenbart EP 0 240 349 A2. Durch Einsatz einer speziellen Siliconemulsion, die nach dem Trocknen ein Elastomer bildet, werden Stylingschäume erhalten, die das Haar sowohl pflegen, als auch festigen. Diese Stylingmittel erlauben jedoch keine Remodellierbarkeit der Fasern und werden durch einfaches Waschen der Fasern mit Wasser oder Shampoonieren wieder aus den Fasern ausgespült, so dass keine dauerhafte Verformung erzielt wird.

Aufgabe der vorliegenden Erfindung war es daher, ein Mittel zur Verfügung zu stellen, dessen Verwendung in einer guten Festigerleistung resultiert, d.h. den behandelten Fasern starken Halt verleiht, die Remodellierbarkeit der Fasern erlaubt, ohne dass der Halt dabei verloren geht, und überdies eine schonende dauerhafte Verformung ermöglicht.

Es wurde nunmehr überraschenderweise gefunden, dass dies durch Verwendung von Stylingmitteln, enthaltend spezielle wässrige Silicondispersionen erreicht werden kann.

Ein erster Gegenstand der vorliegenden Erfindung ist daher die Verwendung eines Mittels, enthaltend in einem kosmetisch akzeptablen Träger mindestens eine wässrige Silicondispersion, wobei die wässrige Silicondispersion
A) 10 bis 70 Gew.-% mindestens eines alpha,omega-(Dihydroxy)-polydiorganosiloxans,
B) 0,01 bis 10 Gew.-% eines hydroxylierten Siliconharzes, das pro Molekül mindestens zwei unterschiedliche Einheiten aufweist, ausgewählt unter Einheiten der Formeln R₃SiO_{0,5} (M), R₂SiO (D), RSiO_{1,5} (T) und SiO₂ (Q), worin R für einen Alkylrest mit 1 bis 6 Kohlenstoffatomen, einen Vinylrest oder einen 3,3,3-Trifluorpropylrest steht, und der Gehalt an Hydroxylgruppen zwischen 0,1 und 10 Gew.-% liegt,
C) 0,1 bis 20 Gew.-% mindestens eines anionischen oder nichtionischen Tensids,
D) 0,001 bis 1 Gew.-% mindestens einer katalytischen Metallverbindung,
E) 0 bis 10 Gew.-% mindestens eines Silazans der Formel H₃Si-(NH-SiH₂)ₙ-NH-SiH₃, wobei n für eine ganze Zahl von 0 bis 20 steht,
F) 0 bis 70 Gew.-% mindestens eines mineralischen Füllstoffs, und
G) 1 bis 40 Gew.-% Wasser
enthält, und wobei die wässrige Silicondispersion in dem Mittel in einer solchen Menge vorliegt, dass die Gesamtmenge der Bestandteile A) bis F), bezogen auf das gesamte Mittel, 0,1 bis 10 Gew.-% beträgt, zur remodellierbaren Verformung keratinischer Fasern.

Silicondispersionen, welche die Bestandteile (A), (B), (C), (D) und (G) enthalten, sind aus der EP 0 410 899 B1 bzw. EP 0 410 899 A1 bekannt. Diese finden vornehmlich zur Herstellung von Dichtungsmassen und Anstrichfarbe Verwendung. Daneben ist auch der Einsatz als Überzugmaterial für pharmazeutische Produkte und in kosmetischen Mitteln genannt. Es findet sich jedoch keinerlei Hinweis darauf, dass die erfindungsgemäß beanspruchte Verwendung von Mitteln, welche die zuvor angeführten Silicondispersionen enthalten zur remodellierbaren Verformung keratinischer Fasern führt.

Vorzugsweise enthalten die erfindungsgemäß verwendeten Mittel eine wässrige Silicondispersion, die den Bestandteil A) in einer Menge von 30 bis 40 Gew.-%, den Bestandteil B) in einer Menge von 0,1 bis 2 Gew.-%, den Bestandteil C) in einer Menge von 0,5 bis 5 Gew.-%, den Bestandteil D) in einer Menge von 0,05 bis 0,2 Gew.-%, den Bestandteil E) in einer Menge von 0,5 bis 5 Gew.-%, den Bestandteil F) in einer Menge von 25 bis 50 Gew.-% und/oder den Bestandteil G) in einer Menge von 5 bis 20 Gew.-% enthält.

Besonders bevorzugt wird eine wässrige Silicondispersion verwendet, die
A) 30 bis 40 Gew.-% mindestens eines alpha,omega-(Dihydroxy)-polydiorganosiloxans,
B) 0,1 bis 2 Gew.-% eines hydroxylierten Siliconharzes, das pro Molekül mindestens zwei unterschiedliche Einheiten aufweist, ausgewählt unter Einheiten der Formeln R₃SiO_{0,5} (M), R₂SiO (D), RSiO_{1,5} (T) und SiO₂ (Q), worin R für einen Alkylrest mit 1 bis 6 Kohlenstoffatomen, einen Vinylrest oder einen 3,3,3-Trifluorpropylrest steht, und der Gehalt an Hydroxylgruppen zwischen 0,1 und 10 Gew.-% liegt,
C) 0,5 bis 5 Gew.-% mindestens eines anionischen oder nichtionischen Tensids,
D) 0,05 bis 0,2 Gew.-% mindestens einer katalytischen Metallverbindung,
E) 0,5 bis 5 Gew.-% mindestens eines Silazans der Formel H₃Si-(NH-SiH₂)ₙ-NH-SiH₃, wobei n für eine ganze Zahl von 0 bis 20 steht,
F) 25 bis 50 Gew.-% mindestens eines mineralischen Füllstoffs, und
G) 5 bis 20 Gew.-% Wasser
enthält.

Als Bestandteil A) sind alpha,omega-(Dihydroxy)-polydiorganosiloxane bevorzugt, die eine Emulsion vom Typ Öl-in-Wasser mit einer dynamischen Viskosität von mindestens 50.000 mPa s bei 25°C ausbilden, wobei die Emulsion durch ein anionisches oder nichtionisches Tensid C) stabilisiert ist.

Als Bestandteil C) finden vorzugsweise die anionischen Alkylbenzolsulfonsäuren und deren Salze, insbesondere Dodecylbenzolsulfonsäure und dessen Alkalimetallsalze, und die nichtionischen Alkylphenolpolyoxyethylene Verwendung.

Als katalytische Metallverbindung D) kommen vorzugsweise Zinnverbindungen und Titanverbindungen oder deren Salze zum Einsatz.

Der mineralische Füllstoff F) ist vorzugsweise ausgewählt aus Calciumcarbonat und hochdisperser Kieselsäure.

Ein besonders ausgewogenes Eigenschaftsspektrum weisen erfindungsgemäß verwendete Mittel auf, die die wässrige Silicondispersion in einer solchen Menge enthalten, dass die Gesamtmenge der Bestandteile A) bis F), bezogen auf das gesamte Mittel, 2 bis 5 Gew.-% beträgt. Bei der erfindungsgemäßen Verwendung dieser Mittel lässt sich besonders hoher Halt bei gleichzeitig ausgezeichneter Remodellierbarkeit erreichen.

Vorzugsweise werden solche Silicondispersionen eingesetzt, die nach dem Trocknen auf der Faser einen Siliconfilm ergeben, der nicht bereits bei einer einmaligen Behandlung mit Wasser oder einem handelsüblichen Shampoo nahezu oder völlig rückstandsfrei aus den behandelten keratinischen Fasern entfernt werden. Unter einer nahezu oder völlig rückstandsfreien Entfernung wird dabei verstanden, dass sich die gewaschenen und anschließend getrockneten Fasern weder klebrig anfühlen, noch eine im Vergleich zu einer Referenzfaser, die nicht mit dem erfindungsgemäß verwendeten Mittel behandelt wurde, verbesserte Haltbarkeit einer mechanischen Verformung aufweist. Der Silikonfilm verbleibt also trotz Wäsche in ausreichender Menge auf den keratinischen Fasern, um auch nach der Wäsche den Fasern Halt zu geben und ein erneutes Formen der Fasern zu erlauben ohne dass weitere Hilfsmittel auf die Fasern aufgebracht werden müssten. Bevorzugt kommen Silicondispersionen zum Einsatz, die Siliconfilme ergeben, die auch eine mehrmalige Wäsche der Fasern, vorzugsweise mindestens 2 Wäschen, besonders bevorzugt mindestens 4 Wäschen überstehen. Auf diese Weise erlaubt die erfindungsgemäße Verwendung eine langanhaltende, im Extremfall sogar permanente Verformung bzw. Verformbarkeit der behandelten Fasern.

Vorzugsweise enthalten die erfindungsgemäß verwendeten Mittel demnach Silicondispersionen, die Siliconfilme ergeben, die auch nach zweimaligem Spülen der Fasern mit Wasser einer Temperatur von 30°C für jeweils 2 Minuten in den Fasern verbleiben, wobei das Wasser pro Spülvorgang in einer solchen Menge eingesetzt wird, dass das Gewichtsverhältnis von Fasern zu Wasser 1 : 10 beträgt.

Die in den erfindungsgemäß verwendeten Mitteln eingesetzten wässrigen Silicondispersionen lassen sich herstellen, indem zunächst alpha,omega-(Dihydroxy)-polydiorganosiloxan A), hydroxyliertes Siliconharz B) und Tensid C) vermischt und anschließend mit Wasser G) versetzt wird. Im Anschluss erfolgt nacheinander die Zugabe von Füllstoff F), Silazan E) und katalytischer Metallverbindung D).

Die erfindungsgemäß verwendeten Mittel lassen sich im einfachsten Fall durch Verdünnen der wässrigen Silicondispersion mit Wasser und/oder weiteren kosmetisch akzeptablen Lösungsmitteln auf die gewünschte Konzentration erhalten. Weitere Bestandteile der erfindungsgemäß verwendeten Mittel werden in bekannter Weise in die Formulierungen eingearbeitet.

Die erfindungsgemäß verwendeten Mittel können neben der Silicondispersion weiterhin mindestens ein filmbildendes und/oder festigendes Polymer enthalten. Allerdings kann auf den Zusatz solcher weiteren filmbildenden und/oder festigenden Polymere auch verzichtet werden.

Die Gesamtmenge an filmbildenden und/oder festigenden Polymeren sollte 0,1 bis 20 Gew.-% betragen, vorzugsweise 0,5 bis 15 Gew.-%, besonders bevorzugt von 1,0 bis 10 Gew.-%.

Diese filmbildenden und/oder festigenden Polymere können sowohl permanent als auch temporär kationisch, anionisch, nichtionisch oder amphoter sein. Bei der Verwendung von mindestens zwei filmbildenden und/oder festigenden Polymeren können diese selbstverständlich unterschiedliche Ladungen aufweisen. Erfindungsgemäß bevorzugt kann es sein, wenn ein ionisches filmbildendes und/oder festigendes Polymer mit einem amphoteren und/oder nichtionischem filmbildenden und/oder festigenden Polymer gemeinsam verwendet wird. Auch die Verwendung mindestens zweier gegensätzlich geladener filmbildender und/oder festigender Polymere ist bevorzugt. In letzterem Falle kann eine besondere Ausführungsform wiederum zusätzlich mindestens ein weiteres amphoteres und/oder nichtionisches filmbildendes und/oder festigendes Polymer enthalten.

Da Polymere häufig multifunktional sind, können deren Funktionen nicht immer klar und eindeutig voneinander abgegrenzt werden. Insbesondere gilt dies für filmbildende und festigende Polymere. Dennoch sollen beispielhaft einige filmbildende Polymere beschrieben werden. Es wird an dieser Stelle jedoch explizit darauf verwiesen, dass im Rahmen der vorliegenden Erfindung sowohl filmbildende als auch festigende Polymere wesentlich sind. Da beide Eigenschaften auch nicht völlig unabhängig voneinander sind, werden unter dem Begriff "festigende Polymere" auch immer "filmbildende Polymere" verstanden und umgekehrt.

Zu den bevorzugten Eigenschaften der filmbildenden Polymeren zählt die Filmbildung. Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Alkohol oder Wasser/Alkohol-Gemischen besitzen, um in dem erfindungsgemäß verwendeten Mittel in vollständig gelöster Form vorzulegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden. Die filmbildenden Polymere können dabei sowohl anionisch, amphoter, nicht-ionisch, permanent kationisch oder temporär kationisch geladen sein.

Geeignete synthetische, filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C₁- bis C₇-Alkylgruppen, besonders bevorzugt C₁- bis C₃-Alkylgruppen sind.

Beispielhaft seien genannt Homopolymere des Vinylcaprolactams, des Vinylpyrrolidons oder des N-Vinylformamids. Weitere geeignete synthetische filmbildende, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine^{®} P 191 von der Firma CHEM-Y, Emmerich, oder Sepigel^{®} 305 von der Firma Seppic vertrieben werden; Polyvinylalkohole, die beispielsweise unter den Handelsbezeichnungen Elvanol^{®} von Du Pont oder Vinol^{®} 523/540 von der Firma Air Products vertrieben werden sowie Polyethylenglykol/Polypropylenglykol-Copolymere, die beispielsweise, unter den Handelsbezeichnungen Ucon^{®} der Union Carbide vertrieben werden.

Geeignete natürliche filmbildende Polymere sind z.B. Cellulosederivate, z. B. Hydroxypropylcellulose mit einem Molekulargewicht von 30.000 bis 50.000 g/mol, welche beispielsweise unter der Handelsbezeichnung Nisso SI^{®} von der Firma Lehmann & Voss, Hamburg, vertrieben wird.

Festigende Polymere tragen zum Halt und/oder zum Aufbau des Haarvolumens und der Haarfülle der Gesamtfrisur bei. Diese sogenannten festigenden Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Substanzen, welche dem Haar weiterhin hydrophobe Eigenschaften verleihen, sind hierbei bevorzugt, weil sie die Tendenz des Haares, Feuchtigkeit, also Wasser, zu absorbieren, verringern. Dadurch wird das schlaffe Herunterhängen der Haarsträhnen vermindert und somit ein langanhaltender Frisurenaufbau und -erhalt gewährleistet. Als Testmethode hierfür wird häufig der sogenannte curl-retention - Test angewendet. Diese polymeren Substanzen können weiterhin erfolgreich in leave-on und rinse-off Haarkuren oder Shampoos eingearbeitet werden. Da Polymere häufig multifunktional sind, das heißt mehrere anwendungstechnisch erwünschte Wirkungen zeigen, finden sich zahlreiche Polymere in mehreren auf die Wirkungsweise eingeteilten Gruppen, so auch im CTFA Handbuch. Wegen der Bedeutung gerade der festigenden Polymere sollen diese daher explizit in Form ihrer INCI - Namen aufgelistet werden. In dieser Liste der erfindungsgemäß bevorzugt zu verwendenden Polymere finden sich somit selbstverständlich gerade auch die genannten filmbildenden Polymere wieder.

Beispiele für gebräuchliche filmbildende, festigende Polymere sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/ Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilyl-carbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate.

Vorzugsweise enthält das erfindungsgemäß verwendete Mittel mindestens ein filmbildendes und/oder festigendes Polymer ausgewählt aus
- Aminomethylpropanolsalzen von Copolymeren des Allylmethacrylats mit einem oder mehreren Monomeren ausgewählt aus Acrylsäure, Methacrylsäure, Acrylsäureester und Methacrylsäureester,
- Vinylpyrrolidon-Vinylacetat-Copolymeren,
- Vinylpyrrolidon-Vinylcaprolactam-Dimethylaminopropylacrylamid-Copolymeren,
- Copolymeren des Octylacrylamids mit t-Butylaminoethylmethacrylat und zwei oder mehr Monomeren ausgewählt aus Acrylsäure, Methacrylsäure, Acrylsäureester und Methacrylsäureester, und
- Copolymeren der C₁₋₂-Alkylsuccinate mit Hydroxyalkylacrylaten und einem oder mehreren Monomeren ausgewählt aus Acrylsäure, Methacrylsäure, Acrylsäureester und Methacrylsäureester.

Entsprechende filmbildende und/oder festigende Polymere sind kommerziell erhältlich. Vorzugsweise handelt es sich bei den genannten Acrylsäureestern und Methacrylsäureestern um C₁-C₁₂-Alkylacrylate und C₁-C₁₂-Alkylmethacrylate, besonders bevorzugt um Methylacrylat, Ethylacrylat, Propylacrylat, Methylmethacrylat, Ethylmethacrylat, Propylmethacrylat und deren Mischungen.

Als Aminomethylpropanolsalz von Copolymeren des Allylmethacrylats mit einem oder mehreren Monomeren ausgewählt aus Acrylsäure, Methacrylsäure, Acrylsäureester und Methacrylsäureester, wird vorzugsweise das Copolymer mit der INCI-Bezeichnung AMP-Acrylates/Allyl Methacrylate Copolymer eingesetzt, das von der Firma Noveon unter der Bezeichnung Fixate™ G-100 vertrieben wird. Das erfindungsgemäß verwendete Mittel enthält dieses Copolymer ganz besonders bevorzugt.

Ein bevorzugtes Vinylpyrrolidon-Vinylacetat-Copolymer ist das PVP/VA Copolymer 60-40 W (INCI-Bezeichnung: VP/VA Copolymer, Aqua, Laurtrimonium Chloride).

Als Vinylpyrrolidon-Vinylcaprolactam/Dimethylaminopropylacrylamid-Copolymer wird vorzugsweise das von ISP unter der Bezeichnung Aquaflex SF 40 erhältliche Copolymer mit der INCI-Bezeichnung VP/Vinyl Caprolactam/DMAPA Acrylates Copolymer eingesetzt.

Ein bevorzugtes Copolymer des Octylacrylamids mit t-Butylaminoethylmethacrylat und zwei oder mehr Monomeren ausgewählt aus Acrylsäure, Methacrylsäure, Acrylsäureester und Methacrylsäureester, ist das von National Starch unter der Bezeichnung Amphomer^{®} erhältliche Copolymer mit der INCI-Bezeichnung Octylacrylamide/Acrylates Butylaminoethyl Methacrylates Copolymer.

Als Copolymer der C₁₋₂-Alkylsuccinate mit Hydroxyalkylacrylaten und einem oder mehreren Monomeren ausgewählt aus Acrylsäure, Methacrylsäure, Acrylsäureester und Methacrylsäureester, ist das von ISP unter der Bezeichnung Allianz™ LT 120 erhältliche Copolymer mit der INCI-Bezeichnung Acrylates/C1-2 Succinates/Hydroxyacrylates Copolymer bevorzugt.

Die erfindungsgemäß verwendeten Mittel können weiterhin pflegende Substanzen enthalten. Geeignet sind beispielsweise Proteinhydrolysate und/oder deren Derivate.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch ß-Aminosäuren und deren Derivate wie ß-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben.

Besonders interessant ist der Einsatz von Seiden-Proteinhydrolysaten. Unter Seide versteht man die Fasern des Kokons des Maulbeer-Seidenspinners (Bombyx mori L.). Die Rohseidenfaser besteht aus einem Doppelfaden Fibroin. Als Kittsubstanz hält Sericin diesen Doppelfaden zusammen. Seide besteht zu 70 - 80 Gew.% aus Fibroin, 19 - 28 Gew.% Sericin, 0,5 - 1 Gew.% aus Fett und 0,5 - 1 Gew.% aus Farbstoffen und mineralischen Bestandteilen.

Proteinhydrolysate pflanzlichen Ursprungs, z. B. Soja-, Mandel-, Erbsen-, Kartoffel- und Weizenproteinhydrolysate, sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans - Isomere, Diastereomere und chirale Isomere.
Erfindungsgemäß ist es auch möglich, eine Mischung aus mehreren Proteinhydrolysaten einzusetzen.

Die Proteinhydrolysate sind in den erfindungsgemäß verwendeten Mitteln beispielsweise in Konzentrationen von 0,01 Gew.% bis zu 20 Gew.%, vorzugsweise von 0,05 Gew.% bis zu 15 Gew.% und ganz besonders bevorzugt in Mengen von 0,05 Gew.% bis zu 5 Gew.%, jeweils bezogen auf die gesamte Anwendungszubereitung enthalten.

Als Pflegestoff eignen sich auch kationische Tenside.

Erfindungsgemäß bevorzugt sind kationische Tenside vom Typ der quartären Ammoniumverbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäß verwendeten Mitteln bevorzugt in Mengen von 0,05 bis 10 Gew.-%, bezogen auf die gesamte Anwendungszubereitung, enthalten. Mengen von 0,1 bis 5 Gew.-% sind besonders bevorzugt.

Als Pflegestoff eignen sich ebenfalls pflegende Polymere. An dieser Stelle sei darauf hingewiesen, dass einige der im Folgenden genannten pflegenden Polymere auch filmbildende und/oder festigende Eigenschaften aufweisen und sich daher auch bei der Beschreibung geeigneter filmbildender und/oder festigender Polymere wieder finden können.

Eine erste Gruppe der pflegenden Polymere sind die kationischen Polymere. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des erfindungsgemäß verwendeten Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I),

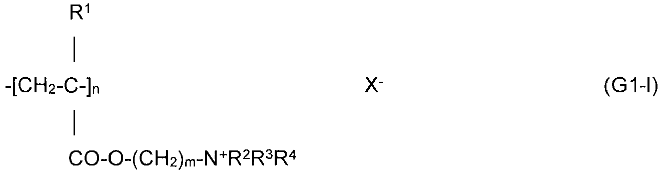

in der R¹= -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
R¹ steht für eine Methylgruppe
R², R³ und R⁴ stehen für Methylgruppen
m hat den Wert 2.

Als physiologisch verträgliche Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich. Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quartären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silikon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäß einsetzbare kationische Polymere sind die so genannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugt eingesetzte kationische Polymere sind kationische Cellulose-Derivate und Chitosan und dessen Derivate, insbesondere die Handelsprodukte Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quartären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Ganz besonders bevorzugt sind die kationischen Proteinhydrolysate und -derivate auf pflanzlicher Basis.

Bevorzugt eingesetzte amphotere Polymere sind solche Polymerisate, die sich im wesentlichen zusammensetzen aus
(a) Monomeren mit quartären Ammoniumgruppen der allgemeinen Formel (II),

   R¹-CH=CR²-CO-Z-(CₙH₂ₙ)-N⁽⁺⁾R³R⁴R⁵ A⁽⁻⁾ (II)

   in der R¹ und R² unabhängig voneinander stehen für Wasserstoff oder eine Methylgruppe und R³, R⁴ und R⁵ jeweils unabhängig voneinander für eine Alkylgruppe mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (III),

   R⁶-CH=CR⁷-COOH (III)

   in denen R⁶ und R⁷ unabhängig voneinander für Wasserstoff oder eine Methylgruppe stehen.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyltrimethyl-ammoniumchlorid ist ein besonders bevorzugtes Monomeres (a). Als Monomeres (b) für die genannten Polymerisate wird bevorzugt Acrylsäure verwendet.

Die erfindungsgemäß verwendeten Mittel enthalten die pflegenden, kationischen oder amphoteren Polymere in bevorzugter Weise in einer Menge von 0,01 bis 5 Gew.-%, insbesondere in einer Menge von 0,1 bis 2 Gew.-%, jeweils bezogen auf die gesamte Anwendungszubereitung.

Als Pflegestoff kann das erfindungsgemäß verwendete Mittel weiterhin mindestens ein Vitamin, ein Provitamin, eine Vitaminvorstufe und/oder eines derer Derivate enthalten.

Dabei sind erfindungsgemäß solche Vitamine, Pro-Vitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Mittel enthalten die Vitamin A-Komponente bevorzugt in Mengen von 0,05-1 Gew.-%, bezogen auf die gesamte Anwendungszubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf die gesamte Anwendungszubereitung, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäß verwendeten Mitteln bevorzugt in Mengen von 0,05 - 10 Gew.-%, bezogen auf die gesamte Anwendungszubereitung, enthalten. Mengen von 0,1 - 5 Gew.-% sind besonders bevorzugt.
- Vitamin B₆ (Pyridoxin sowie Pyridoxamin und Pyridoxal). Die genannten Verbindungen des Vitamin B₆-Typs sind in den erfindungsgemäß verwendeten Mitteln bevorzugt in Mengen von 0,01 - 5 Gew.-%, bezogen auf die gesamte Anwendungszubereitung, enthalten. Mengen von 0,05 - 1 Gew.-% sind besonders bevorzugt.

Vitamin C (Ascorbinsäure). Vitamin C wird in den erfindungsgemäß verwendeten Mitteln bevorzugt in Mengen von 0,1 bis 3 Gew.-%, bezogen auf die gesamte Anwendungszubereitung eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,05-1 Gew.-%, bezogen auf die gesamte Anwendungszubereitung, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäß verwendeten Mitteln bevorzugt in Mengen von 0,0001 bis 1,0 Gew.-%, insbesondere in Mengen von 0,001 bis 0,01 Gew.-%, jeweils bezogen auf die gesamte Anwendungszubereitung enthalten.

Bevorzugt enthalten die erfindungsgemäß verwendeten Mittel Vitamine, Provitamine und Vitaminvorstufen aus den Gruppen A, B, C, E und H.

Panthenol, Pantolacton, Pyridoxin und seine Derivate sowie Nicotinsäureamid und Biotin sind besonders bevorzugt.

Ganz besonders bevorzugt wird als Pflegestoff dieser Gruppe D-Panthenol eingesetzt. Dieser Pflegestoff zeichnet sich durch eine hervorragende Pflegewirkung aus, wobei zudem die vorteilhaften Eigenschaften der erfindungsgemäß verwendeten Mittel hinsichtlich Halt und Remodellierbarkeit nicht negativ beeinflusst werden.

Als Pflegestoff können die erfindungsgemäß verwendeten Mittel weiterhin mindestens einen Pflanzenextrakt enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel. Ganz besonders geeignet sind die Extrakte aus Grünem Tee, Mandel, Aloe Vera, Kokosnuss, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäß verwendeten Mitteln Mischungen aus mehreren, insbesondere aus zwei, verschiedenen Pflanzenextrakten einzusetzen.

Als Pflegestoff eignen sich weiterhin Ectoin oder Ectoinderivate, Allantoin, Taurin und/oder Bisabolol.

Erfindungsgemäß werden unter dem Begriff "Ectoin und Ectoinderivate" Verbindungen der Formel (IV) 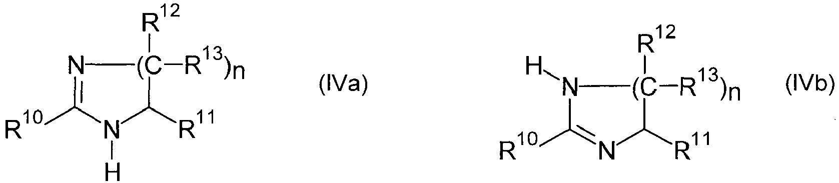 und/oder deren physiologisch verträglichen Salze und/oder isomeren oder stereoisomeren Formen verstanden, wobei
R¹⁰ steht für ein Wasserstoffatom, einen verzweigten oder unverzweigten C₁ - C₄-Alkylrest oder einen C₂ - C₄-Hydroxyalkylrest,
R¹¹ steht für ein Wasserstoffatom, eine Gruppierung -COOR¹⁴ oder eine Gruppierung -CO(NH)R¹⁴, wobei R¹⁴ für ein Wasserstoffatom, einen C₁ - C₄-Alkylrest, einen Aminosäurerest, einen Dipeptid- oder einen Tripeptidrest stehen kann,
R¹² und R¹³ stehen unabhängig voneinander für ein Wasserstoffatom, einen C₁ - C₄-Alkylrest oder eine Hydroxygruppe mit der Maßgabe, dass nicht beide Reste gleichzeitig für eine Hydroxygruppe stehen dürfen, und
n steht für eine ganze Zahl von 1 bis 3.

Geeignete physiologisch verträgliche Salze der allgemeinen Verbindungen gemäß der Formel (IVa) oder (IVb) sind beispielsweise die Alkali-, Erdalkali-, Ammonium-, Triethylamin- oder Tris-(2-hydroxyethyl)aminsalze sowie solche, die sich aus der Umsetzung von Verbindungen gemäß der Formel (IVa) oder (IVb) mit anorganischen und organischen Säuren wie Salzsäure, Phosphorsäure, Schwefelsäure, verzweigten oder unverzweigten, substituierten oder unsubstituierten (beispielsweise durch eine oder mehrere Hydroxygruppen) C₁ - C₄- Mono- oder Dicarbonsäuren, aromatische Carbonsäuren und Sulfonsäuren wie Essigsäure, Citronensäure, Benzoesäure, Maleinsäure, Fumarsäure, Weinsäure und p-Toluolsulfonsäure ergeben. Beispiele für besonders bevorzugte physiologisch verträgliche Salze sind die Na-, K-, Mg- und Ca- und Ammoniumsalze der Verbindungen gemäß der Formel (IVa) oder (IVb), sowie die Salze, die sich durch Umsetzung von Verbindungen gemäß der Formel (IVa) oder (IVb) mit Salzsäure, Essigsäure, Citronensäure und Benzoesäure ergeben.

Unter isomeren oder stereoisomeren Formen der Verbindungen gemäß Formel (IVa) oder (IVb) werden erfindungsgemäß alle auftretenden optischen Isomere, Diastereomere, Racemate, Zwitterionen, Kationen oder Gemische davon verstanden.

Unter dem Begriff Aminosäure werden die stereoisomeren Formen, z.B. D- und L-Formen, folgender Verbindungen verstanden:
Asparagin, Arginin, Asparaginsäure, Glutamin, Glutaminsäure, β-Alanin, γ-Aminobutyrat, N_{ε}-Acetyllysin, Ns-Acetylornitin, N_{γ}-Acetyldiaminobutyrat, N_{α}-Acetyldiaminobutyrat, Histidin, Isoleucin, Leucin, Methionin, Phenylalanin, Serin, Threonin und Tyrosin.
L-Aminosäuren sind bevorzugt. Aminosäurereste leiten sich von den entsprechenden Aminosäuren ab. Die folgenden Aminosäurereste sind bevorzugt:
Gly, Ala, Ser, Thr, Val, β-Ala, γ-Aminobutyrat, Asp, Glu, Asn, Aln, N_{ε}-Acetyllysin, N_{δ}-Acetylornithin, N_{y}-Acetyldiaminobutyrat, N_{α}-Acetyldiaminobutyrat.

Die Kurzschreibweise der Aminosäuren erfolgte nach der allgemein üblichen Schreibweise. Die Di- oder Tripeptidreste sind in ihrer chemischen Natur nach Säureamide und zerfallen bei der Hydrolyse in 2 oder 3 Aminosäuren. Die Aminosäuren in dem Di- oder Tripeptidrest sind durch Amidbindungen miteinander verbunden.

Beispiele für C₁ - C₄-Alkylgruppen in den Verbindungen der Formel (IV) sind Methyl, Ethyl, n-Propyl, Isopropyl, n-Butyl, Isobutyl und tert.-Butyl. Bevorzugte Alkylgruppen sind Methyl und Ethyl, Methyl ist eine besonders bevorzugte Alkylgruppe. Bevorzugte C₂ - C₄-Hydroxyalkylgruppen sind die Gruppen 2-Hydroxyethyl, 3-Hydroxypropyl oder 4-Hydroxybutyl; 2-Hydroxyethyl ist eine besonders bevorzugte Hydroxyalkylgruppe.

Die erfindungsgemäß verwendeten Mittel enthalten diese Pflegestoffe bevorzugt in Mengen von 0,001 bis 2, insbesondere von 0,01 bis 0,5 Gew.-%, jeweils bezogen auf die gesamte Anwendungszubereitung.

Auch Mono- bzw. Oligosaccharide können als Pflegestoff in den erfindungsgemäß verwendeten Mitteln eingesetzt werden.

Es können sowohl Monosaccharide als auch Oligosaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist.

Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside.

Da die eingesetzten Mono- bzw. Oligosaccharide üblicherweise aus natürlichen Rohstoffen wie Stärke gewonnen werden, weisen sie in der Regel die diesen Rohstoffen entsprechenden Konfigurationen auf (z.B. D-Glucose, D-Fructose und D-Galactose).

Die Mono- bzw. Oligosaccharide sind in den erfindungsgemäß verwendeten Haarbehandlungsmitteln bevorzugt in einer Menge von 0,1 bis 8 Gew.-%, insbesondere bevorzugt 1 bis 5 Gew.-%, bezogen auf die gesamte Anwendungszubereitung, enthalten.

Das erfindungsgemäß verwendete Mittel kann weiterhin mindestens ein Lipid als Pflegestoff enthalten.

Erfindungsgemäß geeignete Lipide sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV^{®} vertrieben.

Die erfindungsgemäß verwendeten Mittel enthalten die Lipide bevorzugt in Mengen von 0,01 - 10 Gew.-%, insbesondere 0,1 - 5 Gew.-%, bezogen auf die gesamte Anwendungszubereitung. Weiterhin sind als Pflegestoff Ölkörper geeignet.

Zu den natürlichen und synthetischen kosmetischen Ölkörpern sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyln-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®}OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I), 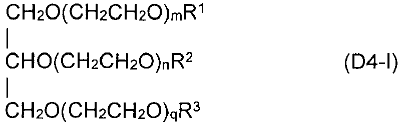 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäß verwendeten Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Das erfindungsgemäß verwendete Mittel kann überdies ein Enzym als Pflegestoff enthalten. Erfindungsgemäß besonders bevorzugte Enzyme sind ausgewählt aus einer Gruppe, die gebildet wird aus Proteasen, Lipasen, Transglutaminase, Oxidasen und Peroxidasen.

Auch Perlenextrakte sind als Pflegestoff geeignet.

Perlen von Muscheln bestehen im Wesentlichen aus anorganischen und organischen Calciumsalzen, Spurenelementen und Proteinen. Perlen lassen sich auf einfache Weise aus kultivierten Muscheln gewinnen. Die Kultivierung der Muscheln kann sowohl in Süßwasser als auch in Meereswasser erfolgen. Dies kann sich auf die Inhaltsstoffe der Perlen auswirken. Erfindungsgemäß bevorzugt ist ein Perlenextrakt, welcher von in Meeres- bzw. Salzwasser kultivierten Muscheln stammt. Die Perlen bestehen zu einem großen Teil aus Aragonit (Calciumcarbonat), Conchiolin und einem Albuminoid. Letztere Bestandteile sind Proteine. Weiterhin sind in Perlen noch Magnesium- und Natriumsalze, anorganische Siliciumverbindungen sowie Phosphate enthalten.

Zur Herstellung des Perlenextraktes werden die Perlen pulverisiert. Danach werden die pulverisierten Perlen mit den üblichen Methoden extrahiert. Als Extraktionsmittel zur Herstellung der Perlenextrakte können Wasser, Alkohole sowie deren Mischungen verwendet werden. Unter Wasser sind dabei sowohl demineralisiertes Wasser, als auch Meereswasser zu verstehen. Unter den Alkoholen sind dabei niedere Alkohole wie Ethanol und Isopropanol, insbesondere aber mehrwertige Alkohole wie Glycerin, Diglycerin, Triglycerin, Polyglycerin, Ethylenglykol, Propylenglykol und Butylenglykol, sowohl als alleiniges Extraktionsmittel als auch in Mischung mit demineralisiertem Wasser oder Meereswasser, bevorzugt. Perlenextrakte auf Basis von Wasser/Glyceringemischen haben sich als besonders geeignet erwiesen. Je nach Extraktionsbedingungen können die Perlenproteine (Conchiloin und Albuminoid) weitestgehend in nativem Zustand oder bereits teilweise oder weitestgehend als Proteinhydrolysate vorliegen. Bevorzugt ist ein Perlenextrakt, in welchem Conchiolin und Albuminoid bereits teilweise hydrolysiert vorliegen. Die wesentlichen Aminosäuren dieser Proteine sind Glutaminsäure, Serin, Alanin, Glycin, Asparaginsäure und Phenylalanin. In einer weiteren besonders bevorzugten Ausgestaltung kann es vorteilhaft sein, wenn der Perlenextrakt zusätzlich mit mindestens einer oder mehreren dieser Aminosäuren angereichert wird. In der bevorzugtesten Ausführungsform ist der Perlenextrakt angereichert mit Glutaminsäure, Serin und Leucin. Weiterhin findet sich je nach Extraktionsbedingungen, insbesondere in Abhängigkeit von der Wahl des Extraktionsmittels ein mehr oder weniger großer Anteil an Mineralien und Spurenelementen im Extrakt wieder. Ein bevorzugter Extrakt enthält organische und/oder anorganische Calciumsalze sowie Magnesium- und Natriumsalze, anorganische Siliciumverbindungen und/oder Phosphate. Ein ganz besonders bevorzugter Perlenextrakt enthält mindestens 75 %; bevorzugt 85 %, besonders bevorzugt 90 % und ganz besonders bevorzugt 95 % aller Inhaltsstoffe der natürlich vorkommenden Perlen. Beispiele für erfindungsgemäß einsetzbare Perlenextrakte sind die Handelsprodukte Pearl Protein Extract BG^{®} oder Crodarom^{®} Pearl.

Die zuvor beschriebenen Perlenextrakte sind vorzugsweise in einer Menge von mindestens 0,01 bis zu 20 Gew.-% enthalten. Bevorzugt werden Mengen des Extraktes von 0,01 bis zu 10 Gew.%, ganz besonders bevorzugt Mengen von 0,01 bis 5 Gew.% bezogen auf das gesamte Mittel verwendet.

Obwohl jeder der genannten Pflegestoffe für sich alleine bereits ein zufriedenstellendes Resultat ergibt, sind im Rahmen der vorliegenden Erfindung auch alle Ausführungsformen umfasst, in denen das erfindungsgemäß verwendete Mittel mehrere Pflegestoffe auch aus verschiedenen Gruppen enthält.

Einige der genannten Pflegestoffe, beispielsweise die oben genannten Ölkörper, können den Halt herabsetzen, der mit der erfindungsgemäßen Verwendung der Mittel erzielt werden kann. Die Pflegestoffe müssen demnach sorgfältig auf die übrigen Bestandteile der Mittel, insbesondere die Silicondispersion, sowie gegebenenfalls eingesetzte filmbildende und/oder festigende Polymere abgestimmt werden. Art bzw. Konzentration dieser Inhaltsstoffe werden so gewählt, dass der Halt, der mit der erfindungsgemäßen Verwendung der Mittel erzielt werden kann, noch die gewünschte Stärke aufweist. Geeignete Kombinationen lassen sich leicht durch einfache Vorversuche ermitteln.

Das erfindungsgemäß verwendete Mittel liegt in einem kosmetisch akzeptablen Träger vor. Dabei handelt es sich bevorzugt um ein wässriges, ein alkoholisches oder ein wässrigalkoholisches Medium mit vorzugsweise mindestens 10 Gewichtsprozent Wasser bezogen auf die gesamte Zubereitung. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent bevorzugt von 1 bis 10 Gewichtsprozent bezogen auf die gesamte Zubereitung enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol, Diethylenglykol und Propylenglykol in einer Menge bis 30 Gewichtsprozent bezogen auf die gesamte Zubereitung.

Die Konfektionierung kann beispielsweise in Form von Cremes, Emulsionen, Gelen oder auch tensidhaltigen schäumenden Lösungen oder andere Zubereitungen erfolgen, die für die Anwendung auf dem Haar geeignet sind. Die Zubereitungen weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C sofern nichts anderes vermerkt ist.

Das erfindungsgemäß verwendete Mittel kann in den bekannten Anwendungsformen vorliegen. Beispielsweise lässt sich das Mittel auf übliche Weise als Aerosolhaarspray, Pumpspray, Haargel, Haarwachs oder Schaumfestiger konfektionieren.

Soll das erfindungsgemäß verwendete Mittel in Form eines Aerosolsprays oder Aerosolschaumfestigers angewendet werden, so wird das Mittel in üblicher Art und Weise in einen druckbeständigen Behälter gefüllt und mit einem Treibmittel versetzt. Der Behälter wird schließlich verschlossen und mit einer geeigneten Sprühvorrichtung versehen.

Als Treibmittel eignen sich die für diesen Zweck üblicherweise eingesetzten Verbindungen, wie Dialkylether, Alkane, N₂O, CO₂ oder Luft. Bevorzugt sind Dimethylether und Alkane mit 3 bis 5 Kohlenstoffatomen, wie Propan, n-Butan, iso-Butan, n-Pentan und iso-Pentan; besonders bevorzugt sind Dimethylether, n-Butan, Propan und deren Mischungen.

Durch Zugabe eines UV-Filters können sowohl die erfindungsgemäß verwendeten Mittel selbst, als auch die behandelten Fasern vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Beispiele für erfindungsgemäß verwendbare UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul^{®}M 40, Uvasorb^{®}MET, Neo Heliopan^{®}BB, Eusolex^{®}4360), 2-Phenylbenzimidazol-5-sulfonsäure und deren Kalium-, Natrium- und Triethanolaminsalze (Phenylbenzimidazole sulfonic acid; Parsol^{®}HS; Neo Heliopan^{®}Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parsol^{®}1789, Eusolex^{®}9020), α-(2-Oxoborn-3-yliden)-toluol-4-sulfonsäure und deren Salze, ethoxylierte 4-Aminobenzoesäure-ethylester (PEG-25 PABA; Uvinul^{®}P 25), 4-Dimethylaminobenzoesäure-2-ethylhexylester (Octyl Dimethyl PABA; Uvasorb^{®}DMO, Escalol^{®}507, Eusolex^{®}6007), Salicylsäure-2-ethylhexylester (Octyl Salicylat; Escalol^{®}587, Neo Heliopan^{®}OS, Uvinul^{®}O18), 4-Methoxyzimtsäure-isopentylester (Isoamyl p-Methoxycinnamate; Neo Heliopan^{®}E 1000), 4-Methoxyzimtsäure-2-ethylhexyl-ester (Octyl Methoxycinnamate; Parsol^{®}MCX, Escalol^{®}557, Neo Heliopan^{®}AV), 2-Hydroxy-4-methoxybenzophenon-5-sulfonsäure und deren Natriumsalz (Benzophenone-4; Uvinul^{®}MS 40; Uvasorb^{®}S 5), 3-(4'-Methylbenzyliden)-D,L-Campher (4-Methylbenzylidene camphor; Parsol^{®}5000, Eusolex^{®}6300), 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon (Benzophenone-1; Uvasorb^{®}20 H, Uvinul^{®}400), 1,1'-Diphenylacrylonitrilsäure-2-ethylhexyl-ester (Octocrylene; Eusolex^{®}OCR, Neo Heliopan^{®}Type 303, Uvinul^{®}N 539 SG), o-Aminobenzoesäure-menthylester (Menthyl Anthranilate; Neo Heliopan^{®}MA), 2,2',4,4'-Tetrahydroxybenzophenon (Benzophenone-2; Uvinul^{®}D-50), 2,2'-Dihydroxy-4,4'-dimethoxybenzophenon (Benzophenone-6), 2,2'-Dihydroxy-4,4'-dimethoxybenzophenon-5-natriumsulfonat und 2-Cyano-3,3-diphenylacrylsäure-2'-ethylhexylester. Bevorzugt sind 4-Amino-benzoesäure, N,N,N-Trimethyl-4-(2-oxoborn-3-ylidenmethyl)anilin-methylsulfat, 3,3,5-Trimethyl-cyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz, 3-(4'-Methylbenzyliden)-D,L-Campher, 3-Benzyliden-campher, 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamid. Erfindungsgemäß ganz besonders bevorzugt sind 2-Hydroxy-4-methoxy-benzophenon, 2-Phenylbenzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4'-Methylbenzyliden)-D,L-Campher.

Bevorzugt sind solche UV-Filter, deren molarer Extinktionskoeffizient am Absorptionsmaximum oberhalb von 15 000, insbesondere oberhalb von 20 000, liegt.

Bei strukturell ähnlichen UV-Filtern weist in vielen Fällen die wasserunlösliche Verbindung die höhere Wirkung gegenüber solchen wasserlöslichen Verbindungen auf, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein. Die Verwendung wasserunlöslicher UV-Filter kann daher erfindungsgemäß bevorzugt sein.

Gemäß einer weiteren Ausführungsform der Erfindung sind solche UV-Filter bevorzugt, die eine kationische Gruppe, insbesondere eine quartäre Ammoniumgruppe, aufweisen.

Diese UV-Filter weisen die allgemeine Struktur U - Q auf.

Der Strukturteil U steht dabei für eine UV-Strahlen absorbierende Gruppe. Diese Gruppe kann sich im Prinzip von den bekannten, im Kosmetikbereich einsetzbaren, oben genannten UV-Filtern ableiten, in dem eine Gruppe, in der Regel ein Wasserstoffatom, des UV-Filters durch eine kationische Gruppe Q, insbesondere mit einer quartären Aminofunktion, ersetzt wird.

Verbindungen, von denen sich der Strukturteil U ableiten kann, sind beispielsweise
- substituierte Benzophenone,
- p-Aminobenzoesäureester,
- Diphenylacrylsäureester,
- Zimtsäureester,
- Salicylsäureester,
- Benzimidazole und
- o-Aminobenzoesäureester.

Strukturteile U, die sich vom Zimtsäureamid oder vom N,N-Dimethylaminobenzoesäureamid ableiten, sind erfindungsgemäß bevorzugt.

Die Strukturteile U können prinzipiell so gewählt werden, dass das Absorptionsmaximum der UV-Filter sowohl im UVA(315-400 nm)-, als auch im UVB(280-315nm)- oder im UVC(<280 nm)-Bereich liegen kann. UV-Filter mit einem Absorptionsmaximum im UVB-Bereich, insbesondere im Bereich von etwa 280 bis etwa 300 nm, sind besonders bevorzugt.

Weiterhin wird der Strukturteil U, auch in Abhängigkeit von Strukturteil Q, bevorzugt so gewählt, dass der molare Extinktionskoeffizient des UV-Filters am Absorptionsmaximum oberhalb von 15 000, insbesondere oberhalb von 20 000, liegt.

Der Strukturteil Q enthält als kationische Gruppe bevorzugt eine quartäre Ammoniumgruppe. Diese quartäre Ammoniumgruppe kann prinzipiell direkt mit dem Strukturteil U verbunden sein, so dass der Strukturteil U einen der vier Substituenten des positiv geladenen Stickstoffatomes darstellt. Bevorzugt ist jedoch einer der vier Substituenten am positiv geladenen Stickstoffatom eine Gruppe, insbesondere eine Alkylengruppe mit 2 bis 6 Kohlenstoffatomen, die als Verbindung zwischen dem Strukturteil U und dem positiv geladenen Stickstoffatom fungiert.

Vorteilhafterweise hat die Gruppe Q die allgemeine Struktur -(CH₂)_{X}-N⁺R¹R²R³ X⁻, in der x steht für eine ganze Zahl von 1 bis 4, R¹ und R² unabhängig voneinander stehen für C₁₋₄-Alkylgruppen, R³ steht für eine C₁₋₂₂-Alkylgruppe oder eine Benzylgruppe und X- für ein physiologisch verträgliches Anion. Im Rahmen dieser allgemeinen Struktur steht x bevorzugt für die Zahl 3, R¹ und R² jeweils für eine Methylgruppe und R³ entweder für eine Methylgruppe oder eine gesättigte oder ungesättigte, lineare oder verzweigte Kohlenwasserstoffkette mit 8 bis 22, insbesondere 10 bis 18, Kohlenstoffatomen.

Physiologisch verträgliche Anionen sind beispielsweise anorganische Anionen wie Halogenide, insbesondere Chlorid, Bromid und Fluorid, Sulfationen und Phosphationen sowie organische Anionen wie Lactat, Citrat, Acetat, Tartrat, Methosulfat und Tosylat.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyldimethylaminobenzamidopropyl-dimethylammoniumtosylat (Escalol^{®} HP 610).

Selbstverständlich umfasst die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In einer besonderen Ausführungsform enthält das erfindungsgemäß verwendete Mittel weiterhin einen oder mehrere direktziehende Farbstoffe. Dies ermöglicht, dass bei der erfindungsgemäßen Verwendung des Mittels die behandelte keratinische Faser nicht nur strukturiert, sondern zugleich auch gefärbt wird.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 11, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Bevorzugt werden kationische direktziehende Farbstoffe eingesetzt. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Die erfindungsgemäß verwendeten Mittel gemäß dieser Ausführungsform enthalten die direktziehenden Farbstoffe bevorzugt in einer Menge von 0,001 bis 20 Gew.-%, bezogen auf das gesamte Mittel.

Weiterhin können die erfindungsgemäß verwendeten Mittel auch in der Natur vorkommende Farbstoffe enthalten, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Es ist nicht erforderlich, dass die direktziehenden Farbstoffe jeweils einheitliche Verbindungen darstellen. Vielmehr können in den erfindungsgemäß verwendeten Mitteln, bedingt durch die Herstellungsverfahren für die einzelnen Farbstoffe, in untergeordneten Mengen noch weitere Komponenten enthalten sein, soweit diese nicht das Stylingergebnis nachteilig beeinflussen oder aus anderen Gründen, z.B. toxikologischen, ausgeschlossen werden müssen.

Die erfindungsgemäß verwendeten Mittel können neben den genannten Komponenten weiterhin alle für solche Zubereitungen bekannten Wirk-, Zusatz- und Hilfsstoffe enthalten.

Weitere Wirk-, Hilfs- und Zusatzstoffe, sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- Parfümöle, Dimethylisosorbid und Cyclodextrine,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Substanzen zur Einstellung des pH-Wertes, wie beispielsweise übliche Säuren, insbesondere Genußsäuren und Basen,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Antioxidantien.

Bezüglich weiterer fakultativer Komponenten sowie die eingesetzten Mengen dieser Komponenten wird ausdrücklich auf die dem Fachmann bekannten einschlägigen Handbücher verwiesen.

## Patentansprüche

1. Verwendung eines Mittels, enthaltend in einem kosmetisch akzeptablen Träger mindestens eine wässrige Silicondispersion, **dadurch gekennzeichnet, dass** die wässrige Silicondispersion
A) 10 bis 70 Gew.-% mindestens eines alpha,omega-(Dihydroxy)-polydiorganosiloxans,
B) 0,01 bis 10 Gew.-% eines hydroxylierten Siliconharzes, das pro Molekül mindestens zwei unterschiedliche Einheiten aufweist, ausgewählt unter Einheiten der Formeln R₃SiO_{0,5} (M), R₂SiO (D), RSiO_{1,5} (T) und SiO₂ (Q), worin R für einen Alkylrest mit 1 bis 6 Kohlenstoffatomen, einen Vinylrest oder einen 3,3,3-Trifluorpropylrest steht, und der Gehalt an Hydroxylgruppen zwischen 0,1 und 10 Gew.-% liegt,
C) 0,1 bis 20 Gew.-% mindestens eines anionischen oder nichtionischen Tensids,
D) 0,001 bis 1 Gew.-% mindestens einer katalytischen Metallverbindung,
E) 0 bis 10 Gew.-% mindestens eines Silazans der Formel H₃Si-(NH-SiH₂)ₙ-NH-SiH₃, wobei n für eine ganze Zahl von 0 bis 20 steht,
F) 0 bis 70 Gew.-% mindestens eines mineralischen Füllstoffs, und
G) 1 bis 40 Gew.-% Wasser
enthält, und die wässrige Silicondispersion in dem Mittel in einer solchen Menge vorliegt, dass die Gesamtmenge der Bestandteile A) bis F), bezogen auf das gesamte Mittel, 0,1 bis 10 Gew.-% beträgt,
zur remodellierbaren Verformung keratinischer Fasern.

2. Verwendung nach Anspruch 1, **dadurch gekennzeichnet, dass** die wässrige Silicondispersion den Bestandteil A) in einer Menge von 30 bis 40 Gew.-% enthält.

3. Verwendung nach einem der Ansprüche 1 bis 2, **dadurch gekennzeichnet, dass** die wässrige Silicondispersion den Bestandteil B) in einer Menge von 0,1 bis 2 Gew.-% enthält.

4. Verwendung nach einem der Ansprüche 1 bis 3, **dadurch gekennzeichnet, dass** die wässrige Silicondispersion den Bestandteil C) in einer Menge von 0,5 bis 5 Gew.-% enthält.

5. Verwendung nach einem der Ansprüche 1 bis 4, **dadurch gekennzeichnet, dass** die wässrige Silicondispersion den Bestandteil D) in einer Menge von 0,05 bis 0,2 Gew.-% enthält.

6. Verwendung nach einem der Ansprüche 1 bis 5, **dadurch gekennzeichnet, dass** die wässrige Silicondispersion den Bestandteil E) in einer Menge von 0,5 bis 5 Gew.-% enthält.

7. Verwendung nach einem der Ansprüche 1 bis 6, **dadurch gekennzeichnet, dass** die wässrige Silicondispersion den Bestandteil F) in einer Menge von 25 bis 50 Gew.-% enthält.

8. Verwendung nach einem der Ansprüche 1 bis 7, **dadurch gekennzeichnet, dass** die wässrige Silicondispersion den Bestandteil G) in einer Menge von 5 bis 20 Gew.-% enthält.

9. Verwendung nach einem der Ansprüche 1 bis 8, **dadurch gekennzeichnet, dass** die wässrige Silicondispersion
A) 30 bis 40 Gew.-% mindestens eines alpha,omega-(Dihydroxy)-polydiorganosiloxans,
B) 0,1 bis 2 Gew.-% eines hydroxylierten Siliconharzes, das pro Molekül mindestens zwei unterschiedliche Einheiten aufweist, ausgewählt unter Einheiten der Formeln R₃SiO_{0,5} (M), R₂SiO (D), RSiO_{1,5} (T) und SiO₂ (Q), worin R für einen Alkylrest mit 1 bis 6 Kohlenstoffatomen, einen Vinylrest oder einen 3,3,3-Trifluorpropylrest steht, und der Gehalt an Hydroxylgruppen zwischen 0,1 und 10 Gew.-% liegt,
C) 0,5 bis 5 Gew.-% mindestens eines anionischen oder nichtionischen Tensids,
D) 0,05 bis 0,2 Gew.-% mindestens einer katalytischen Metallverbindung,
E) 0,5 bis 5 Gew.-% mindestens eines Silazans der Formel H₃Si-(NH-SiH₂)ₙ-NH-SiH₃, wobei n für eine ganze Zahl von 0 bis 20 steht,
F) 25 bis 50 Gew.-% mindestens eines mineralischen Füllstoffs, und
G) 5 bis 20 Gew.-% Wasser
enthält.

10. Verwendung nach einem der Ansprüche 1 bis 9, **dadurch gekennzeichnet, dass** die katalytische Metallverbindung D) ausgewählt ist aus Zinnverbindungen und Titanverbindungen oder deren Salzen.

11. Verwendung nach einem der Ansprüche 1 bis 10, **dadurch gekennzeichnet, dass** der mineralische Füllstoff F) ausgewählt ist aus Calciumcarbonat und hochdisperser Kieselsäure.

12. Verwendung nach einem der Ansprüche 1 bis 11, **dadurch gekennzeichnet, dass** es zusätzlich mindestens ein filmbildendes und/oder festigendes Polymer in einer Menge von 0,1 bis 20 Gew.-%, vorzugsweise 0,5 bis 15 Gew.-%, besonders bevorzugt von 1,0 bis 10 Gew.-% enthält.

13. Verwendung nach einem der Ansprüche 1 bis 12, **dadurch gekennzeichnet, dass** es sich bei dem kosmetisch akzeptablen Träger um ein wässriges, ein alkoholisches oder ein wässrigalkoholisches Medium handelt.

14. Verwendung nach einem der Ansprüche 1 bis 13, **dadurch gekennzeichnet, dass** die wässrige Silicondispersion in dem Mittel in einer solchen Menge vorliegt, dass die Gesamtmenge der Bestandteile A) bis F), bezogen auf das gesamte Mittel, 2 bis 5 Gew.-% beträgt.

## Claims

1. Use of an agent containing, in a cosmetically acceptable vehicle, at least one aqueous silicone dispersion, **characterized in that** the aqueous silicone dispersion contains:
A) 10 to 70% by weight of at least one α,ω-dihydroxypolydiorganosiloxane,
B) 0.01 to 10% by weight of a hydroxylated silicone resin containing, per molecule, at least two different units, selected from units of the formulas R₃SiO_{0.5} (M), R₂SiO (D), RSiO_{1.5} (T) and SiO₂ (Q), where R stands for an alkyl radical with 1 to 6 carbon atoms, a vinyl radical or a 3,3,3-trifluoropropyl radical, and the hydroxyl group content is between 0.1 and 10% by weight,
C) 0.1 to 20% by weight of at least one anionic or nonionic surfactant,
D) 0.001 to 1% by weight of at least one catalytic metal compound,
E) 0 to 10% by weight of at least one silazane of the formula H₃Si-(NH-SiH₂)ₙ-NH-SiH₃ where n stands for an integer from 0 to 20,
F) 0 to 70% by weight of at least one mineral filler, and
G) 1 to 40% by weight water,
and the aqueous silicone dispersion is present in the agent in an amount such that the total amount of ingredients A) through F), based on the total agent, amounts to 0.1 to 10% by weight,
for remodelable shaping of keratinic fibers.

2. Use according to claim 1, **characterized in that** the aqueous silicone dispersion contains the ingredient A) in an amount of 30 to 40% by weight.

3. Use according to one of claims 1 to 2, **characterized in that** the aqueous silicone dispersion contains the ingredient B) in an amount of 0.1 to 2% by weight.

4. Use according to one of claims 1 to 3, **characterized in that** the aqueous silicone dispersion contains the ingredient C) in an amount of 0.5 to 5% by weight.

5. Use according to one of claims 1 to 4, **characterized in that** the aqueous silicone dispersion contains the ingredient D) in an amount of 0.05 to 0.2% by weight.

6. Use according to one of claims 1 to 5, **characterized in that** the aqueous silicone dispersion contains the ingredient E) in an amount of 0.5 to 5% by weight.

7. Use according to one of claims 1 to 6, **characterized in that** the aqueous silicone dispersion contains the ingredient F) in an amount of 25 to 50% by weight.

8. Use according to one of claims 1 to 7, **characterized in that** the aqueous silicone dispersion contains the ingredient G) in an amount of 5 to 20% by weight.

9. Use according to one of claims 1 to 8, **characterized in that** the aqueous silicone dispersion contains
A) 30 to 40% by weight of at least one α,ω-dihydroxypolydiorganosiloxane,
B) 0.1 to 2% by weight of a hydroxylated silicone resin containing, per molecule, at least two different units, selected from units of the formulas R₃SiO_{0.5} (M), R₂SiO (D), RSiO_{1.5} (T) and SiO₂ (Q), where R stands for an alkyl radical with 1 to 6 carbon atoms, a vinyl radical or a 3,3,3-trifluoropropyl radical, and the hydroxyl group content is between 0.1 and 10% by weight,
C) 0.5 to 5% by weight of at least one anionic or nonionic surfactant,
D) 0.05 to 0.2% by weight of at least one catalytic metal compound,
E) 0.5 to 5% by weight of at least one silazane of the formula H₃Si-(NH-SiH₂)ₙ-NH-SiH₃, where n stands for an integer from 0 to 20,
F) 25 to 50% by weight of at least one mineral filler, and
G) 5 to 20% by weight water.

10. Use according to one of claims 1 to 9, **characterized in that** the catalytic metal compound D) is selected from tin compounds and titanium compounds or salts thereof.

11. Use according to one of claims 1 to 10, **characterized in that** the mineral filler F) is selected from calcium carbonate and highly dispersed silicic acid.

12. Use according to one of claims 1 to 11, **characterized in that** it additionally contains at least one film-forming and/or setting polymer in an amount of 0.1 to 20% by weight, preferably 0.5 to 15% by weight, especially preferably 1.0 to 10% by weight.

13. Use according to one of claims 1 to 12, **characterized in that** the cosmetically acceptable vehicle is an aqueous, alcoholic or aqueous-alcoholic medium.

14. Use according to one of claims 1 to 13, **characterized in that** the aqueous silicone dispersion is present in the agent in an amount such that the total amount of ingredients A) through F), based on the total agent, is 2 to 5% by weight.

## Revendications

1. Utilisation d'un agent contenant, dans un support cosmétiquement acceptable, au moins une dispersion aqueuse de silicone, **caractérisée en ce que** la dispersion aqueuse de silicone contient
A) 10 à 70% en poids au moins un alpha, oméga-(dihydroxy)-polydiorganosiloxanes,
B) 0,01 à 10% en poids d'une résine silicone hydroxylée comportant par molécule au moins deux unités différentes choisies parmi des unités des formules R₃SiO_{0,5} (M), R₂SiO (D), RSiO_{1,5} (T) et SiO₂ (Q) dans lesquelles R est un radical alkyle comportant 1 à 6 atomes de carbone, un radical vinyle ou un radical 3,3,3-trifluoropropyle, et la teneur en groupes hydroxyle est comprise entre 0,1 et 10% en poids,
C) 0,1 à 20% en poids d'au moins un tensioactif anionique ou non ionique,
D) 0,001% à 1% en poids d'au moins un composé métallique catalytique,
E) 0 à 10% en poids d'au moins un silazane de la formule H₃Si-(NH-SiH₂)ₙ-NH-SiH₃, où n est un nombre entier de 0 à 20,
F) 0 à 70% en poids d'au moins une charge minérale, et
G) 1 à 40% en poids d'eau,
et la dispersion aqueuse de silicone est présente dans l'agent dans une quantité telle que la quantité totale des composants A) à F), par rapport à l'agent total, est de 0,1 à 10% en poids,
pour mettre en forme des fibres de kératine de façon remodelable.

2. Utilisation selon la revendication 1, **caractérisée en ce que** la dispersion aqueuse de silicone contient le composant A) dans une quantité de 30 à 40% en poids.

3. Utilisation selon l'une des revendications 1 à 2, **caractérisée en ce que** la dispersion aqueuse de silicone contient le composant B) dans une quantité de 0,1 à 2% en poids.

4. Utilisation selon l'une des revendications 1 à 3, **caractérisée en ce que** la dispersion aqueuse de silicone contient le composant C) dans une quantité de 0,5 à 5% en poids.

5. Utilisation selon l'une des revendications 1 à 4, **caractérisée en ce que** la dispersion aqueuse de silicone contient le composant D) dans une quantité de 0,05 à 0,2% en poids.

6. Utilisation selon l'une des revendications 1 à 5, **caractérisée en ce que** la dispersion aqueuse de silicone contient le composant E) dans une quantité de 0,5 à 5% en poids.

7. Utilisation selon l'une des revendications 1 à 6, **caractérisée en ce que** la dispersion aqueuse de silicone contient le composant F) dans une quantité de 25 à 50% en poids.

8. Utilisation selon l'une des revendications 1 à 7, **caractérisée en ce que** la dispersion aqueuse de silicone contient le composant G) dans une quantité de 5 à 20% en poids.

9. Utilisation selon l'une des revendications 1 à 8, **caractérisée en ce que** la dispersion aqueuse de silicone contient
A) 30 à 40% en poids d'au moins un alpha, oméga-(dihydroxy)-polydiorganosiloxanes,
B) 0,01 à 2% en poids d'une résine silicone hydroxylée comportant par molécule au moins deux unités différentes choisies parmi des unités des formules R₃SiO_{0,5} (M), R₂SiO (D), RSiO_{1,5} (T) et SiO₂ (Q) dans lesquelles R est un radical alkyle comportant 1 à 6 atomes de carbone, un radical vinyle ou un radical 3,3,3-trifluoropropyle, et la teneur en groupes hydroxyle est comprise entre 0,1 et 10% en poids,
C) 0,5 à 5% en poids d'au moins un tensioactif anionique ou non ionique,
D) 0,005% à 0,2% en poids d'au moins un composé métallique catalytique,
E) 0,5 à 5% en poids d'au moins un silazane de la formule H₃Si-(NH-SiH₂)ₙ-NH-SiH₃, où n est un nombre entier de 0 à 20,
F) 25 à 50% en poids d'au moins une charge minérale, et
G) 5 à 20% en poids d'eau.

10. Utilisation selon l'une des revendications 1 à 9, **caractérisée en ce que** le composé métallique catalytique D) est choisi parmi des composés d'étain et des composés de titane ou de leurs sels.

11. Utilisation selon l'une des revendications 1 à 10, **caractérisée en ce que** la charge minérale F) est choisie parmi le carbonate de calcium et la silice finement divisée.

12. Utilisation selon l'une des revendications 1 à 11, **caractérisée en ce qu'**elle comprend en outre au moins une polymère filmogène et/ou fixant dans une quantité de 0,1 à 20% en poids, de préférence de 0,5 à 15% en poids, de manière plus préférée de 1,0 à 10% en poids.

13. Utilisation selon l'une des revendications 1 à 12, **caractérisée en ce que** le support cosmétiquement acceptable est un milieu aqueux, alcoolique ou hydro-alcoolique.

14. Utilisation selon l'une des revendications 1 à 13, **caractérisée en ce que** la dispersion aqueuse de silicone est présente dans l'agent dans une quantité telle que la quantité totale des composants A) à F), par rapport à l'agent total, est de 2 à 5% en poids.
